(19) Europäisches Patentamt
European Patent Office
Office européen des brevets

(11) Publication number: 0 411 184 B1

(12) EUROPEAN PATENT SPECIFICATION

(45) Date of publication of patent specification: 29.12.93

(51) Int. Cl.5: C07D 307/62, C07D 405/12, C09K 15/06, C09K 15/16, A61K 31/375

(21) Application number: 89114273.9

(22) Date of filing: 02.08.89

The file contains technical information submitted after the application was filed and not included in this specification

# (54) Ascorbic acid derivative.

(43) Date of publication of application:
06.02.91 Bulletin 91/06

(45) Publication of the grant of the patent:
29.12.93 Bulletin 93/52

(84) Designated Contracting States:
AT BE CH DE ES FR GB GR IT LI LU NL SE

(56) References cited:
EP-A- 0 086 554
EP-A- 0 146 121
EP-A- 0 202 589

JOURNAL OF THE CHEMICAL SOCIETY, PERKIN TRANSACTIONS 1, no. 2, February 1986, pages 369-376, London, GB; T. TANAKA et al.: "Tannins and related compounds. Part 37. Isolation and structure elucidation of elaeocarpusin, a novel ellagitannin from Elaeocarpus sylvestris var. Ellipticus"

(73) Proprietor: NIPPON HYPOX LABORATORIES INCORPORATED
1759, Matsugaya
Hachioji-shi
Tokyo 192-03(JP)

(72) Inventor: Satoh, Toshio
57-3, Nagao
Jyoroku-cho
Tokushima-shi Tokushima-ken(JP)
Inventor: Niiro, Yasunori No. 403
2nd Masuoka Bdlg
1-3, Minamisako-hachiban-cho
Tokushima-shi Tokushima-ken(JP)
Inventor: Kakegawa, Hisao
No. 307, City-Corpo. Kimura
3-59, Okihama-higashi
Tokushima-shi Tokushima-ken(JP)
Inventor: Matsumoto, Hitoshi
125-22, Shimofukuman
Hachiman-cho
Tokushima-shi Tokushima-ken(JP)

EP 0 411 184 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CARBOHYDRATE RESEARCH, vol. 102, 1982, pages 302-307, Amsterdam, NL; H.A. PARISH, Jr. et al.: "The reaction of 1,1,-dibromopinacolone with L-ascorbic acid"

CHEMICAL ABSTRACTS, vol. 110, no. 6, 6th February 1989, page 373, abstract no. 44747t, Columbus, Ohio, US; & JP-A-63 190 882 (NIPPON HYPOX K.K.) 08-08-1988

CHEMICAL ABSTRACTS, Vol.72, 1970, abstract No 21324K

J.MED.CHEM., Vol 31, April 1988, p.793-798

(74) Representative: **Hansen, Bernd, Dr.rer.nat. et al**
**Hoffmann, Eitle & Partner**
**Patentanwälte**
**Postfach 81 04 20**
**D-81904 München (DE)**

## Description

BACKGROUND OF THE INVENTION

(1) Field of the Invention

This invention relates to an ascorbic acid derivative, a process for preparing the same and an antioxidant comprising an ascorbic acid derivative.

(2) Prior Art

Ascorbic acid has antioxidant action and is used for the purpose of preventing browning of foods, retaining flavor or freshness of foods or the like.

Ascorbic acid, however, is susceptible to decomposition and sometimes hard to produce the above-mentioned effects over a long period.

J. Chem. Soc. Perkin 1, 1986, pp. 369-376 reveals 3-O-(4-bromophenacyl)-ascorbic acid.

Chem. Abs., vol. 72, 1970, abstract 21324k reveals 3-O-phenacyl-ascorbic acid.

J. Med. Chem., vol. 31, April 1988, pp. 793-798 discloses the antioxidant activity of 3-O-octadecyl-ascorbic acid.

Chem. Abs., vol. 110, 1989, abstract 44747t discloses the use of 3-O-(alkoxycarbonylmethyl)-ascorbic acid as an inhibitor of melanin formation.

EP-A-0 146 121 describes ascorbic acid 2-ethers useful as antioxidant agents for food.

SUMMARY OF THE INVENTION

It is, therefore, a first object of this invention to provide a novel ascorbic acid derivative eliminating the aforementioned disadvantages of the ascorbic acid. It is a second object of this invention to provide a process for preparing the aforesaid novel ascorbic acid derivative. It is further a third object of this invention to provide an antioxidant comprising an ascorbic acid derivative.

The first object of this invention has been achieved by an ascorbic acid derivative represented by the general formula (Ia):

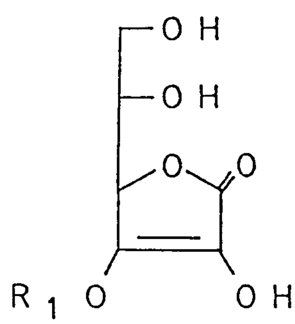

(Ia)

wherein $R_1$ is selected from the group consisting of a heterocyclic ring-containing $(C_1-C_5)$alkyl group represented by general formula:

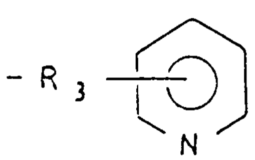 , 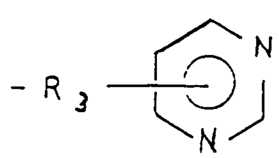 or 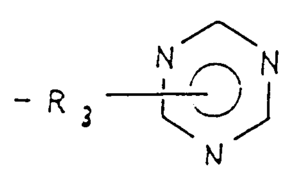

wherein $R_3$ represents a $(C_1-C_5)$alkylene group, and a $(C_7-C_{22})$alkoxycarbonyl-$(C_1-C_5)$alkyl group containing the terminal $(C_7-C_{22})$alkoxy group.

The second object of this invention has been accomplished by a process for preparing an ascorbic acid derivative represented by the general formula (Ia):

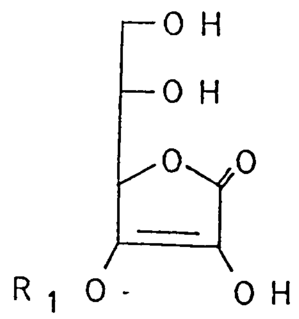

( I a )

wherein $R_1$ is selected from the group consisting of a heterocyclic ring-containing $(C_1\text{-}C_5)$alkyl group represented by general formula:

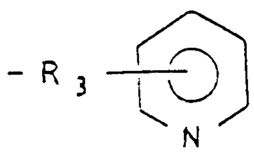 , 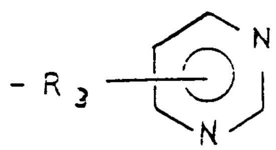 or 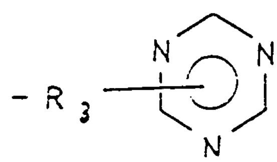

wherein $R_3$ represents a $(C_1\text{-}C_5)$alkylene group, and a $(C_7\text{-}C_{22})$alkoxycarbonyl-$(C_1\text{-}C_5)$alkyl group containing the terminal $(C_7\text{-}C_{22})$alkoxy group, comprising treating a compound represented by the general formula (II):

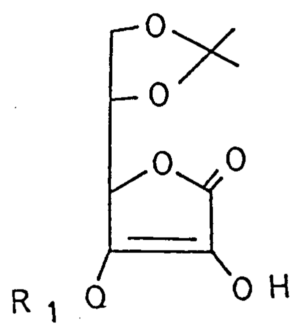

( II )

wherein $R_1$ has the same significance as above, with an acid to cleave the dioxolane ring in said compound and form a vic-glycol group.

Furthermore, the third object of this invention has been achieved by the use of an ascorbic acid derivative represented by the general formula (IA):

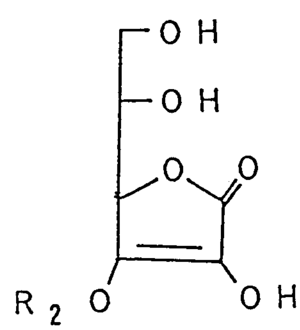

( I A )

wherein $R_2$ is a group selected from the group consisting of a heterocyclic ring-containing $(C_1\text{-}C_5)$alkyl group represented by general formula:

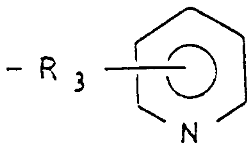 , 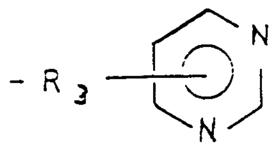 or 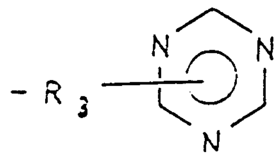

wherein $R_3$ represents a $(C_1\text{-}C_5)$alkylene group, a $(C_1\text{-}C_5)$alkylcarbonyl-$(C_1\text{-}C_5)$alkyl group, a $(C_6\text{-}C_{12})$-arylcarbonyl-$(C_1\text{-}C_5)$alkyl group, a $(C_7\text{-}C_{22})$alkoxycarbonyl-$(C_1\text{-}C_5)$alkyl group containing the terminal $(C_7\text{-}C_{22})$alkoxy group, a $(C_1\text{-}C_{12})$alkyl group, a $(C_7\text{-}C_{12})$aralkyl group and a hydroxycarbonyl-$(C_1\text{-}C_{22})$alkyl group.

The group $R_1$ in the general formula (Ia) representing the novel ascorbic acid derivative of this invention is different in the number of substituent groups defined therein from the group $R_2$ in the general formula (IA) representing the ascorbic acid derivative constituting the antioxidant of this invention.

The novel ascorbic acid derivative of this invention is initially explained hereinafter.

The novel ascorbic acid derivative of this invention is represented by the general formula (Ia):

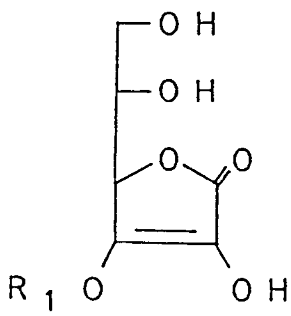

( I a )

wherein $R_1$ is defined as outlined above.

Among the heterocyclic ring-containing $(C_1\text{-}C_5)$alkyl groups particularly preferred are a pyridylmethyl group, a pyrimidylmethyl group and a triazylmethyl group.

Examples of the $(C_7\text{-}C_{22})$alkoxycarbonyl-$(C_1\text{-}C_5)$alkyl groups containing the terminal alkoxy group having at least 7 carbon atoms include groups represented by the general formula:

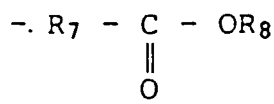

wherein $R_7$ is a $(C_1\text{-}C_5)$alkylene group which may optionally have a branched chain; $R_8$ is a $(C_7\text{-}C_{22})$-alkyl group. A particularly preferred alkoxycarbonylalkyl group is $-CH_2\text{-}CO\text{-}O\text{-}n\text{-}C_{10}H_{21}$.

The novel ascorbic acid derivative of this invention has excellent antioxidant action and can be used as food antioxidants or beautifying and whitening cosmetics or antioxidant for eliminating radicals.

The process for preparing the novel ascorbic acid derivative represented by the above-mentioned general formula (Ia) of this invention is explained hereinafter.

In the process of this invention, a compound represented by the general formula (II):

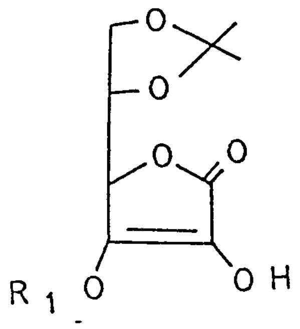

(II)

wherein $R_1$ is as defined above, is used as a starting material.

Such a compound represented by the formula (II) is obtained by ketalizing ascorbic acid according to a conventional method to provide 5,6-O-isopropylideneascrobic acid represented by the formula (III):

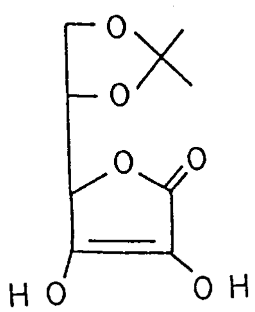

(III)

and further reacting the resulting compound (III) with an organic halide represented by the general formula: $R_1X$ wherein $R_1$ is the same as the group $R_1$ in the general formula (II); X is a halogen atom, to etherify the hydroxyl group at the 3-position of the compound (III). This dehydrohalogenation may be carried out either according to the Williamson reaction or in an organic phase-aqueous phase system using a phase transfer catalyst.

According to the process of this invention, the compound (II) as a starting material is treated with an acid to cleave the dioxolane ring in the above-mentioned compound to form a vic-glycol group. Thereby the desired ascorbic acid derivative represented by the general formula (Ia) is obtained.

Example of the acid used for the reaction include hydrochloric acid, acetic acid, sulfuric acid, p-toluenesulfonic acid, methanesulfonic acid, camphorsulfonic acid and the like. The reaction is preferably carried out in at least one organic solvents selected from methanol, ethanol, dioxane, tetrahydrofuran and 1,2-dimethoxyethane.

The antioxidant of this invention is explained hereinafter.

As explained above, the antioxidant of this invention comprises the ascorbic acid derivative represented by the general formula (IA):

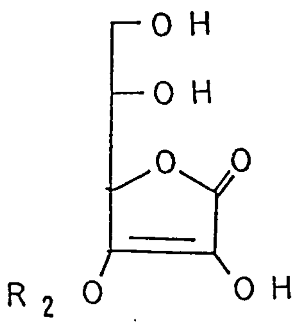

(IA)

The group $R_2$ in the formula is within a wider scope than the group $R_1$ in the general formula (Ia) representing the novel ascorbic acid derivative as described above, and therefore the ascorbic acid derivative represented by the general formula (IA) includes also known compounds.

Thus, the group $R_2$ includes a heterocyclic ring-containing ($C_1$-$C_5$)alkyl group represented by general formula:

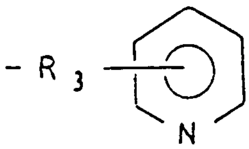
,
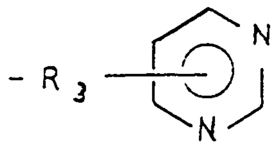
or
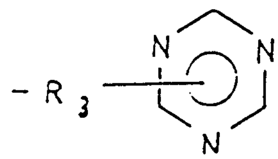

wherein $R_3$ represents a ($C_1$-$C_5$)alkylene group, a ($C_1$-$C_5$)alkylcarbonyl-($C_1$-$C_5$)alkyl group, a ($C_6$-$C_{12}$)-arylcarbonyl-($C_1$-$C_5$)alkyl group, a ($C_7$-$C_{22}$)alkoxycarbonyl-($C_1$-$C_5$)alkyl group, a ($C_1$-$C_{12}$)alkyl group, a ($C_7$-$C_{12}$)aralkyl group and a hydroxycarbonyl-($C_1$-$C_{22}$)alkyl group.

The term "alkyl" in the aforesaid substituent groups means a straight or branched alkyl group.

Examples of the ($C_1$-$C_5$)alkylcarbonyl-($C_1$-$C_5$)alkyl groups include groups represented by the general formula:

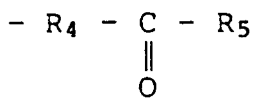

wherein $R_4$ is a ($C_1$-$C_5$)alkylene group; $R_5$ is a ($C_1$-$C_5$)alkyl group. Particularly preferred alkylcarbonylalkyl groups are

$-CH_2-CO-CH_3$, $-CH_2CO-C_2H_5$

Examples of the arylcarbonylalkyl groups include groups represented by the general formula:

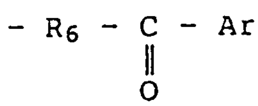

wherein $R_6$ is a ($C_1$-$C_5$)alkylene group; Ar is a ($C_6$-$C_{12}$)aryl group. A particularly preferred arylcarbonylalkyl group is $-CH_2CO-C_6H_5$.

The ascorbic acid derivatives represented by the general formula (IA) have the ability to eliminate radicals and are preferably used as an antioxidant with advantages in better stability than that of ascorbic acid and conventional ascorbic acid derivatives.

[Examples]

Examples of this invention are explained hereinafter.

Preparation example 1 [Preparation of ascorbic acid derivative (IA)]

(1) Synthesis of L-5,6-O-isopropylideneascorbic acid

Ascorbic acid in an amount of 180 g was stirred in 750 mℓ of acetone and warmed to 40 °C. Acetyl chloride in a volume of 20 mℓ was added, and stirring was continued to form a slurry layer.

After 3 hours, the slurry layer was cooled with ice to collect deposited precipitates by filtration. The resulting precipitates were washed with a mixture of cold acetone-n-hexane (3:7) on a funnel and dried with silica gel under reduced pressure.

Recrystallization from acetone was then carried out to provide 190g of L-5,6-O-isopropylidene ascorbic acid (melting point: 206-208°C).

(2) Synthesis of L-5,6-O-isopropylidene-3-O-benzoylmethylascorbic acid

In 30 mℓ of DMSO, was dissolved 4.32 g of the compound obtained in (1). After $NaHCO_3$ in an amount of 1.78 g was added, the resulting solution was stirred at room temperature for 30 minutes. To the solution, was added 4.37 g of phenacyl bromide, and the obtained solution was warmed to 40°C and stirred for 18 hours. After cooling, 80 mℓ of $H_2O$ was added, and pH was adjusted to 5 with 4N hydrochloric acid. The pH-adjusted solution was shaken with ethyl acetate (100 mℓ x 2). Organic layers were combined, washed with water and saturated aqueous sodium chloride, dried over sodium sulfate and concentrated under reduced pressure. The resulting oily substance was subjected to silica gel column chromatography and eluted with a mixture of benzene-ethyl acetate to provide a L-5,6-O-isopropylidene-3-O-benzoylmethylascorbic acid.

(3) Synthesis of L-3-O-benzoylmethylascorbic acid

In 40 mℓ of a mixture of tetrahydrofuranmethanol (3:1), was dissolved 3.3 g of the compound obtained in (2), and 10 mℓ of 2N hydrochloric acid was added to stir the resulting solution at room temperature for 20 hours. The reaction solution was concentrated under reduced pressure, and the residue was recrystallized from ethyl acetate-petroleum ether to afford L-3-O-benzoylmethylascorbic acid (corresponding to compound No. 112 in Table-1).

The resulting compound No. 112 of this invention was used for antioxidant tests described below.

Preparation example 2

[Preparation of ascorbic acid derivative (Ia) and (IA)]

Procedures were followed in the same manner as in Preparation example 1 to provide novel compound No. 110 and known compound No 115 shown in Table-1 mentioned below.

Such compounds were used for the antioxidant tests described below.

Preparation example 3

[Preparation of novel ascorbic acid derivative (Ia)]

(1) Synthesis of L-5,6-isopropylidene-3-O-(3-picoyl) ascorbic acid

In 40 mℓ of distilled water, was dissolved 1.66 g of $NaHCO_3$, and 80 mℓ of methyl ethyl ketone was added. To the resulting solution, was added 4.32 g of the L-5,6-O-isopropylideneascorbic acid obtained in (1) of Preparation example 1. The obtained mixture was stirred, and 3.26 g of 3-picoyl chloride, 1.66 g of $NaHCO_3$ and 1.60 g of tetrabutylammonium bromide were added thereto. The resulting mixture was heated to 70°C and vigorously stirred for 10 hours. The organic layer was separated, and the aqueous layer was adjusted to pH 4 with 4N hydrochloric acid and shaken with 100 mℓ of ethyl acetate. Organic layers were combined, washed with water, dried over sodium sulfate and concentrated under reduced pressure. The resulting residue was subjected to silica gel column chromatography and eluted with a mixture of benzene-ethyl acetate to provide L-5,6-isopropylidene-3-O-(3-picoyl)ascorbic acid.

(2) Synthesis of L-3-O-(3-picoyl) ascorbic acid

Operations were performed in the same manner as in (3) of Preparation example 1, except that the compound obtained in (1) was used. Recrystallization from benzene-petroleum ether was carried out to provide L-3-O-(3-picoyl)ascorbic acid (corresponding to compound No. 113 in Table-1).

The obtained compound No. 113 of this invention was used for the antioxidant tests mentioned below.

Preparation referential example 1

[Preparation of other ascorbic acid derivatives included in ascorbic acid derivative (IA)]

Procedures were followed in the same manner as in Preparation example 1 to provide compound Nos. 101, 102, 103, 104, 105, 106, 107, 108, 109, 111 and 114 indicated in Table-1 mentioned below.

The resulting compounds were also used for the antioxidant tests described below.

Table-1

| Compound No. | $R_1$ | mp | NMR δ value |
|---|---|---|---|
| 101* | $-(CH_2)_{17}CH_3$ | 103°C | 0.85 (3H, m) 1.24 (32H, m) 3.45 (3H, m)<br>4.36 (2H, t) 4.73 (H, s) |
| 102 | $-(CH_2)_{11}CH_3$ | 86-88°C | 0.85 (3H, m) 1.25 (20H, m) 3.35 (3H, m)<br>4.36 (2H, t) 4.73 (H, s) |
| 103 | $-(CH_2)_7CH_3$ | 58-61°C | 0.86 (3H, m) 1.27 (12H, m) 3.50 (3H, m)<br>4.36 (2H, t) 4.73 (H, s) |
| 104 | $-(CH_2)_3CH_3$ | Oily substance | 0.94 (3H, m) 1.60 (4H, m) 3.71 (3H, m)<br>4.48 (2H, t) 4.79 (H, d, 1Hz) |
| 105 | $-(CH_2)_4COOC_2H_5$ | Oily substance | 1.21 (3H, t) 1.76 (4H, m) 2.36 (2H, m)<br>3.70 (3H, m) 4.09 (2H, q) 4.49 (2H, t)<br>4.79 (H, s) |
| 106 | $-(CH_2)_3COOC_2H_5$ | Oily substance | 1.21 (3H, t) 2.17 (2H, m) 2.47 (2H, t)<br>3.70 (3H, m) 4.09 (2H, q) 4.51 (2H, t)<br>4.80 (H, s) |
| 107 | $-(CH_2)_3COOH$ | Oily substance | 2.05 (2H, m) 2.48 (2H, t) 3.70 (3H, m)<br>4.54 (2H, t) 4.80 (H, s) |
| 108 | $CH_3$<br>$-CH_2-COOC_2H_5$ | 141°C | 1.20 (3H, t) 1.48 (2H, d) 3.34 (3H, m)<br>4.15 (2H, q) 4.81 (H, s) 5.31 (H, q) |
| 109 | $-CH_2COO-n-C_4H_9$ | 81°C | 0.91 (3H, t) 1.56 (4H, m) 3.73 (3H, m)<br>4.14 (2H, t) 4.90 (H, d, 2Hz) 5.08 (2H, s) |

* reference compound

- Cont'd -

Table-1 (Cont'd)

| Compound No. | $R_1$ | mp | NMR δ value |
|---|---|---|---|
| 110 | $-CH_2COO-n-C_{10}H_{21}$ | Oily substance | 0.88 (3H, m) 1.25 (16H, m) 3.70 (3H, m) 4.28 (2H, t) 4.90 (H, s) 5.09 (2H, s) |
| 111 | $-CH_2COOC_2H_5$ | Oily substance | 1.29 (3H, t) 3.68 (3H, m) 4.27 (2H, q) 4.87 (H, d, 2Hz) 5.03 (2H, s) |
| 112 | $-CH_2CO$ 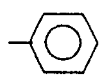 | 92°C | 3.44 (3H, m) 4.92 (H, s) 5.79 (H, d, 7Hz) 7.61 (3H, m) 7.96 (2H, m) |
| 113 | $-CH_2$ 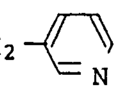 | 154-160°C | 3.69 (3H, m) 4.85 (H, d, 2Hz) 5.61 (2H, s) 7.50 (H, m) 8.0 (H, m) 8.59 (2H, m) |
| 114 | $-(CH_2)_{10}COOH$ | 90°C | 1.32 (16H, m) 2.29 (2H, t) 3.61 (3H, m) 4.48 (2H, t) 4.78 (H, s) |
| 115 | $-CH_2COCH_3$ | 143°C | 2.10 (3H, s) 3.48 (3H, m) 4.84 (H, s) 5.01 (2H, m) |

Test example 1 (Antioxidant action examined by using stable radicals)

Reduction activity of α,α-diphenyl-β-picrylhydrazyl (DPPH) which was a stable free radical was examined according to the M. S. Blois method (Nature, vol. 181, page 1199, 1958) and used as an index to

antioxidant action. Thus, specimens were added to 3 mℓ of a 0.1 mM DPPH solution in ethanol, and absorbance at a wavelength of 517 nm was measured using a spectrophotometer after 20 minutes. The difference in absorbance from the solvent control [0.5% or less of dimethylformamide (DMF)] was taken as the reduction activity.

The 50% radical eliminating concentrations for the test compounds are shown in Table-2.

As can be seen from Table-2, the test compounds were found to have improved antioxidant action. It has been also clarified that the other compound Nos. 102 to 115 have better antioxidant ability than the reference compound No.101 wherein $R_1$ is a long-chain alkyl group.

Table 2

| Compound No. | 50% radical eliminating concentration |
|---|---|
| 101* | $3.2 \times 10^{-5}$ M |
| 102 | 1.8 |
| 103 | 1.9 |
| 104 | 2.3 |
| 105 | 2.0 |
| 106 | 2.1 |
| 107 | 2.7 |
| 108 | 1.9 |
| 109 | 1.8 |
| 110 | 2.3 |
| 111 | 2.2 |
| 112 | 2.3 |
| 113 | 2.3 |
| 114 | 2.4 |
| 115 | 2.2 |

* reference compound.

As detailed above, this invention provides a novel ascorbic acid derivative having excellent antioxidant action and a process for preparing the same.

Furthermore, this invention also provides a novel antioxidant comprising the aforementioned ascorbic acid derivative or other known ascorbic acid derivatives.

## Claims

**Claims for the following Contracting States : AT, BE, CH, DE, FR, GB, GR, IT, LI, LU, NL, SE**

1. An ascorbic acid derivative represented by general formula (Ia):

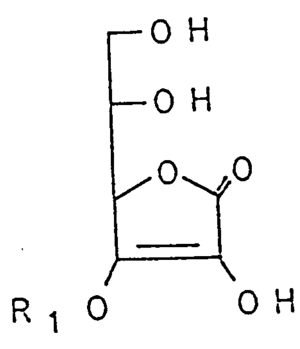

(Ia)

wherein $R_1$ is selected from the group consisting of a heterocyclic ring-containing ($C_1$-$C_5$)alkyl group represented by general formula:

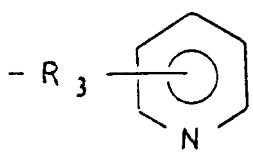 , 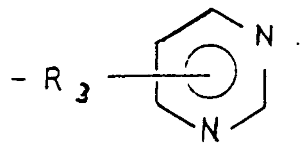 or 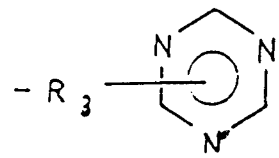

wherein $R_3$ represents a $(C_1-C_5)$alkylene group, and a $(C_7-C_{22})$alkoxycarbonyl-$(C_1-C_5)$alkyl group containing the terminal $(C_7-C_{22})$alkoxy group.

2. An ascorbic acid derivative of Claim 1, wherein said heterocyclic ring-containing $(C_1-C_5)$alkyl group is selected from the group consisting of pyridylmethyl, pyrimidylmethyl and triazylmethyl.

3. An ascorbic acid derivative of Claim 1, wherein said alkoxycarbonylalkyl group is represented by general formula:

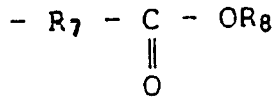

wherein $R_7$ represents a $(C_1-C_5)$alkylene group which may optionally have a branched chain and $R_8$ is a $(C_7-C_{22})$alkyl group.

4. An ascorbic acid derivative of Claim 3, wherein said alkoxycarbonylalkyl group is $-CH_2-COO-n-C_{10}H_{21}$.

5. A process for preparing an ascorbic acid derivative represented by general formula (Ia):

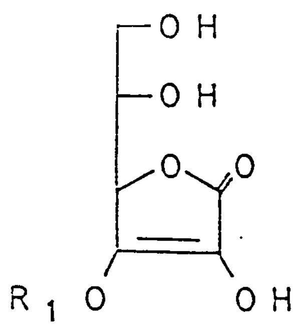

(I a)

wherein $R_1$ is selected from the group consisting of a heterocyclic ring-containing $(C_1-C_5)$alkyl group represented by general formula:

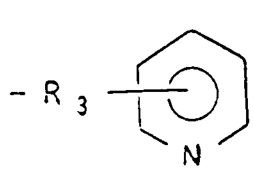 , 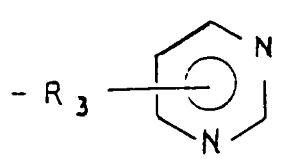 or 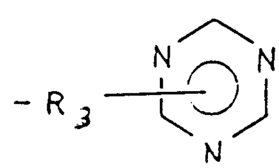

wherein $R_3$ represents a $(C_1-C_5)$alkylene group, and a $(C_7-C_{22})$alkoxycarbonyl-$(C_1-C_5)$alkyl group containing the terminal $(C_7-C_{22})$alkoxy group which comprises treating a compound represented by general formula (II):

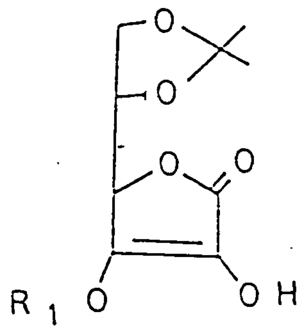

( II )

wherein $R_1$ has the same significance as above, with an acid to cleave the dioxolan ring in said compound and form a vic-glycol group.

**6.** A process of Claim 5, wherein said acid is selected from the group consisting of hydrochloric acid, acetic acid, sulfuric acid, p-toluenesulfonic acid, methanesulfonic acid and camphorsulfonic acid.

**7.** A process of Claim 5, wherein the reaction is conducted in at least one organic solvent selected from the group consisting of methanol, ethanol, dioxane, tetrahydrofuran and 1,2-dimethoxyethane.

**8.** Use of an ascorbic acid derivative represented by general formula (IA):

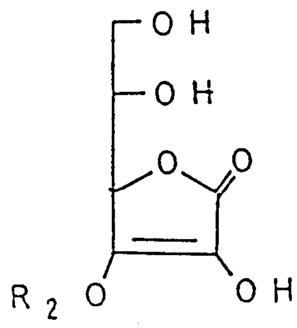

( I A )

wherein $R_2$ is a group selected from the group consisting of a heterocyclic ring-containing $(C_1\text{-}C_5)$alkyl group represented by general formula:

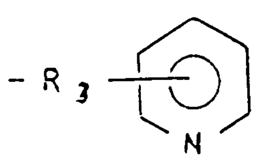 , 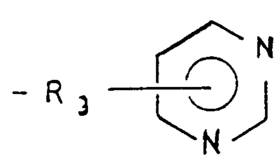 or 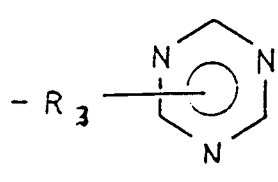

wherein $R_3$ represents a $(C_1\text{-}C_5)$alkylene group, a $(C_1\text{-}C_5)$alkylcarbonyl-$(C_1\text{-}C_5)$alkyl group, a $(C_6\text{-}C_{12})$-arylcarbonyl-$(C_1\text{-}C_5)$alkyl group, a $(C_7\text{-}C_{22})$alkoxycarbonyl-$(C_1\text{-}C_5)$alkyl group containing the terminal $(C_7\text{-}C_{22})$alkoxy group, a $(C_1\text{-}C_{12})$alkyl group, a $(C_7\text{-}C_{12})$aralkyl group and a hydroxycarbonyl-$(C_1\text{-}C_{22})$-alkyl group as a food antioxidant, beautifying and whitening cosmetics or antioxidant for eliminating radicals.

**9.** Use according to Claim 8, wherein said heterocyclic ring-containing $(C_1\text{-}C_5)$alkyl group is selected from the group consisting of pyridylmethyl, pyrimidylmethyl and triazylmethyl.

**10.** Use according to Claim 8, wherein said $(C_1\text{-}C_5)$alkylcarbonyl-$(C_1\text{-}C_5)$alkyl group is represented by the general formula:

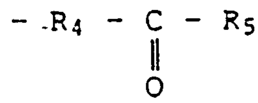

wherein $R_4$ represents a $(C_1\text{-}C_5)$alkylene group and $R_5$ represents a $(C_1\text{-}C_5)$alkyl group.

**11.** Use according to Claim 10, wherein said $(C_1\text{-}C_5)$alkylcarbonyl-$(C_1\text{-}C_5)$alkyl group is selected from the group consisting of $-CH_2CO\text{-}CH_3$ and $-CH_2\text{-}CO\text{-}C_2H_5$.

**12.** Use according to Claim 8, wherein said $(C_6\text{-}C_{12})$arylcarbonyl-$(C_1\text{-}C_5)$alkyl group is represented by general formula:

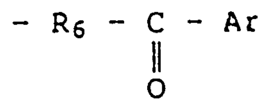

wherein $R_6$ represents a $(C_1\text{-}C_5)$alkylene group and Ar is a $(C_6\text{-}C_{12})$aryl group.

**13.** Use according to Claim 12, wherein said $(C_6\text{-}C_{12})$arylcarbonyl-$(C_1\text{-}C_5)$alkyl group is $-CH_2CO\text{-}C_6H_5$.

**14.** Use according to Claim 8, wherein said $(C_7\text{-}C_{22})$alkoxycarbonyl-$(C_1\text{-}C_5)$alkyl group containing the terminal $(C_7\text{-}C_{22})$alkoxy group is represented by general formula:

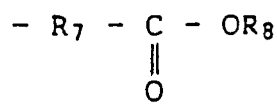

wherein $R_7$ represents a $(C_1\text{-}C_5)$alkylene group which may optionally have a branched chain and $R_8$ is a $(C_7\text{-}C_{22})$alkyl group.

**15.** Use according to Claim 14, wherein $R_8$ is a $(C_7\text{-}C_{22})$alkyl group.

**16.** Use according to Claim 15, wherein said $(C_7\text{-}C_{22})$alkoxycarbonyl-$(C_1\text{-}C_5)$alkyl group is $-CH_2\text{-}COO\text{-}n\text{-}C_{10}H_{21}$.

**17.** Use according to Claim 8, wherein said $(C_1\text{-}C_5)$alkyl group is a straight or branched alkyl group.

**18.** Use according to Claim 8, wherein the alkyl moiety of said hydroxycarbonyl$(C_1\text{-}C_{22})$alkyl group is a straight or branched alkyl group.

**19.** Use according to Claim 8, wherein the alkyl moiety of said $(C_7\text{-}C_{12})$aralkyl group is a straight or branched alkyl group.

**Claims for the following Contracting State : ES**

1. A process for preparing an ascorbic acid derivative represented by general formula (Ia):

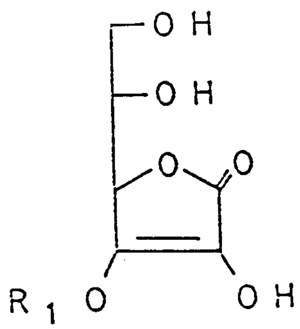

( I a )

wherein $R_1$ is selected from the group consisting of a heterocyclic ring-containing ($C_1$-$C_5$)alkyl group represented by general formula:

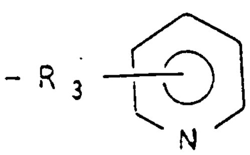
,
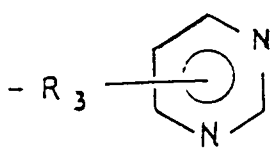
or
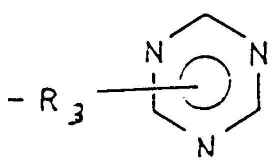

wherein $R_3$ represents a ($C_1$-$C_5$)alkylene group, and a ($C_7$-$C_{22}$)alkoxycarbonyl-($C_1$-$C_5$)alkyl group containing the terminal ($C_7$-$C_{22}$)alkoxy group which comprises treating a compound represented by general formula (II):

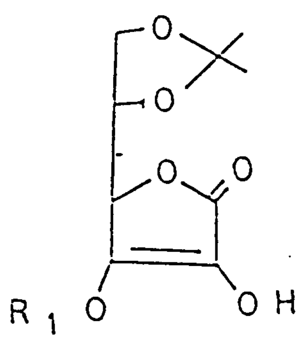

( II )

wherein $R_1$ has the same significance as above, with an acid to cleave the dioxolan ring in said compound and form a vic-glycol group.

2. A process according to Claim 1, wherein said heterocyclic ring-containing ($C_1$-$C_5$)alkyl group is selected from the group consisting of pyridylmethyl, pyrimidylmethyl and triazylmethyl.

3. A process according to Claim 1, wherein said alkoxycarbonylalkyl group is represented by general formula:

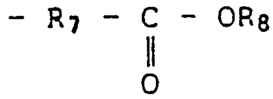

wherein $R_7$ represents a ($C_1$-$C_5$)alkylene group which may optionally have a branched chain and $R_8$ is a ($C_7$-$C_{22}$)alkyl group.

**4.** A process according to Claim 3, wherein said alkoxycarbonylalkyl group is $-CH_2-COO-n-C_{10}H_{21}$.

**5.** A process according to Claim 1, wherein said acid is selected from the group consisting of hydrochloric acid, acetic acid, sulfuric acid, p-toluenesulfonic acid, methanesulfonic acid and camphorsulfonic acid.

**6.** A process according to Claim 1, wherein the reaction is conducted in at least one organic solvent selected from the group consisting of methanol, ethanol, dioxane, tetrahydrofuran and 1,2-dimethoxyethane.

**7.** A process for preparing a composition used as a food antioxidant, as beautifying and whitening cosmetics or as antioxidant for eliminating radicals, wherein an ascorbic acid derivative represented by the general formula (IA):

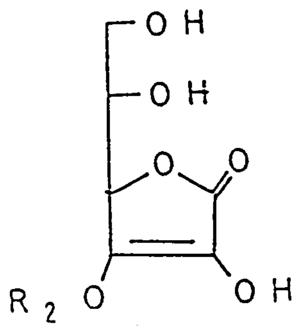

(IA)

wherein $R_2$ is a group selected from the group consisting of a heterocyclic ring-containing ($C_1$-$C_5$)alkyl group represented by general formula:

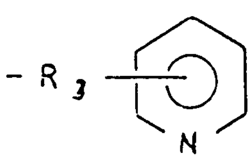
,
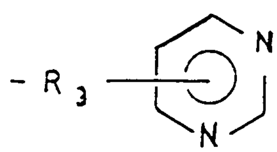
or
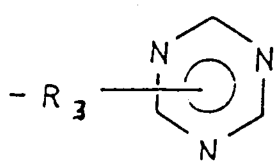

wherein $R_3$ represents a ($C_1$-$C_5$)alkylene group, a ($C_1$-$C_5$)alkylcarbonyl-($C_1$-$C_5$)alkyl group, a ($C_6$-$C_{12}$)-arylcarbonyl-($C_1$-$C_5$)alkyl group, a ($C_7$-$C_{22}$)alkoxycarbonyl-($C_1$-$C_5$)alkyl group containing the terminal ($C_7$-$C_{22}$)alkoxy group, a ($C_1$-$C_{12}$)alkyl group, a ($C_7$-$C_{12}$)aralkyl group and a hydroxycarbonyl-($C_1$-$C_{22}$)-alkyl group is mixed with acceptable additives.

**8.** A process according to Claim 7, wherein said heterocyclic ring-containing ($C_1$-$C_5$)alkyl group is selected from the group consisting of pyridylmethyl, pyrimidylmethyl and triazylmethyl.

**9.** A process according to Claim 7, wherein said ($C_1$-$C_5$)alkylcarbonyl-($C_1$-$C_5$)alkyl group is represented by the general formula:

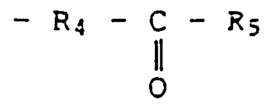

wherein $R_4$ represents a $(C_1$-$C_5)$alkylene group and $R_5$ represents a $(C_1$-$C_5)$alkyl group.

**10.** A process according to Claim 9, wherein said $(C_1$-$C_5)$alkylcarbonyl-$(C_1$-$C_5)$alkyl group is selected from the group consisting of $-CH_2CO-CH_3$ and $-CH_2-CO-C_2H_5$.

**11.** A process according to Claim 7, wherein said $(C_6$-$C_{12})$arylcarbonyl-$(C_1$-$C_5)$alkyl group is represented by general formula:

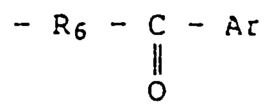

wherein $R_6$ represents a $(C_1$-$C_5)$alkylene group and Ar is a $(C_6$-$C_{12})$aryl group.

**12.** A process according to Claim 11, wherein said $(C_6$-$C_{12})$arylcarbonyl-$(C_1$-$C_5)$alkyl group is $-CH_2CO-C_6H_5$.

**13.** A process according to Claim 7, wherein said $(C_7$-$C_{22})$alkoxycarbonyl-$(C_1$-$C_5)$alkyl group containing the terminal $(C_7$-$C_{22})$alkoxy group is represented by general formula:

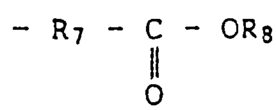

wherein $R_7$ represents a $(C_1$-$C_5)$alkylene group which may optionally have a branched chain and $R_8$ is a $(C_7$-$C_{22})$alkyl group.

**14.** A process according to Claim 13, wherein $R_8$ is a $(C_7$-$C_{22})$alkyl group.

**15.** A process according to Claim 14, wherein said $(C_7$-$C_{22})$alkoxycarbonyl-$(C_1$-$C_5)$alkyl group is $-CH_2-COO-n-C_{10}H_{21}$.

**16.** A process according to Claim 7, wherein said $(C_1$-$C_5)$alkyl group is a straight or branched alkyl group.

**17.** A process according to Claim 7, wherein the alkyl moiety of said hydroxycarbonyl$(C_1$-$C_{22})$alkyl group is a straight or branched alkyl group.

**18.** A process according to Claim 7, wherein the alkyl moiety of said $(C_7$-$C_{12})$aralkyl group is a straight or branched alkyl group.

**Patentansprüche**

**Patentansprüche für folgende Vertragsstaaten : AT, BE, CH, DE, FR, GB, GR, IT, LI, LU, NL, SE**

1. Ein Ascorbinsäurederivat, welches durch die allgemeine Formel (Ia) dargestellt wird:

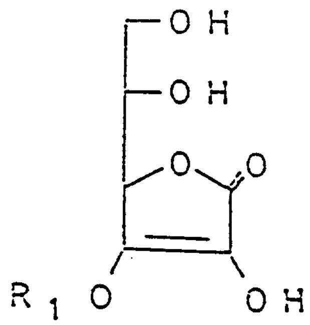

(Ia),

worin $R_1$ ausgewählt ist aus der Gruppe, welche aus einer einen heterocyclischen Ring enthaltenden $(C_1\text{-}C_5)$Alkylgruppe besteht, welche durch die allgemeinen Formeln dargestellt wird:

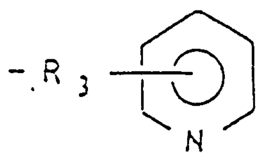 , 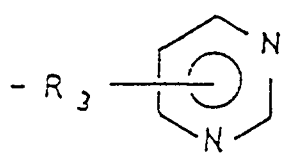 oder 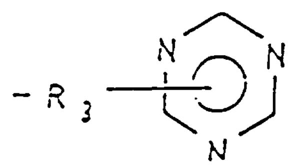 ,

worin $R_3$ eine $(C_1\text{-}C_5)$Alkylengruppe und eine $(C_7\text{-}C_{22})$Alkoxycarbonyl-$(C_1\text{-}C_5)$alkylgruppe darstellt, welche die $(C_7\text{-}C_{22})$Alkoxygruppe endständig enthält.

2. Ein Ascorbinsäurederivat nach Anspruch 1, worin die genannte, einen heterocyclischen Ring enthaltende $(C_1\text{-}C_5)$Alkylgruppe aus einer Gruppe ausgewählt ist, die aus Pyridylmethyl, Pyrimidylmethyl und Triazylmethyl besteht.

3. Ein Ascorbinsäurederivat gemäß Anspruch 1, worin die genannte Alkoxycarbonylalkylgruppe durch die allgemeine Formel dargestellt wird:

$$-R_7-\underset{\underset{O}{\|}}{C}-OR_8\ ,$$

worin $R_7$ eine $(C_1\text{-}C_5)$Alkylengruppe darstellt, welche gegebenenfalls eine verzweigte Kette hat, und $R_8$ eine $(C_7\text{-}C_{22})$Alkylgruppe ist.

4. Ein Ascorbinsäurederivat gemäß Anspruch 3, wobei die genannte Alkoxycarbonylalkylgruppe $-CH_2-COO-n-C_{10}H_{21}$ ist.

5. Ein Verfahren zur Herstellung eines Ascorbinsäurederivates, welches durch die allgemeine Formel (Ia) dargestellt wird:

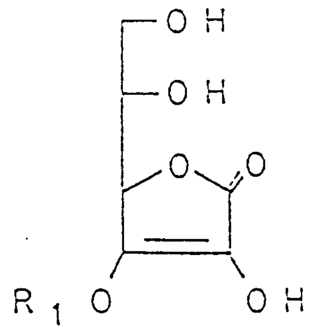

(Ia),

worin $R_1$ ausgewählt ist aus der Gruppe, welche aus einer einen heterocyclischen Ring enthaltenden ($C_1$-$C_5$)Alkylgruppe besteht, welche durch die allgemeinen Formeln dargestellt wird:

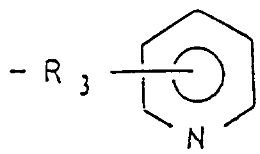 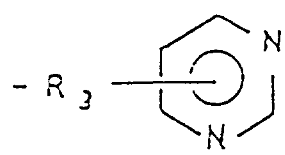 oder 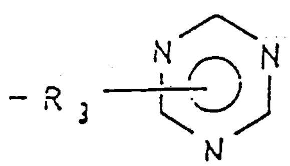 ,

worin $R_3$ eine ($C_1$-$C_5$)Alkylengruppe und eine ($C_7$-$C_{22}$)Alkoxycarbonyl-($C_1$-$C_5$)alkylgruppe darstellt, welche die ($C_7$-$C_{22}$)Alkoxygruppe endständig enthält, wobei dieses Verfahren die Behandlung einer Verbindung, welche durch die allgemeine Formel (II) dargestellt wird:

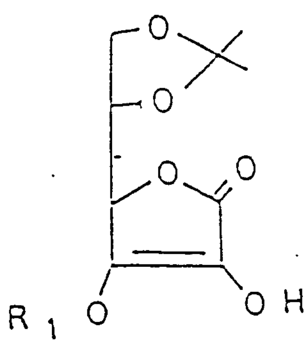

(II),

worin $R_1$ dieselbe Bedeutung wie oben hat, mit einer Säure einschließt, um den Dioxolanring in der genannten Verbindung aufzuspalten und eine vic-Glykolgruppe zu bilden.

**6.** Ein Verfahren gemäß Anspruch 5, wobei die genannte Säure aus einer Gruppe ausgewählt wird, welche aus Salzsäure, Essigsäure, Schwefelsäure, p-Toluolsulfonsäure, Methansulfonsäure und Camphersulfonsäure besteht.

**7.** Ein Verfahren gemäß Anspruch 5, wobei die Reaktion bevorzugt in mindestens einem organischen Lösungsmittel durchgeführt wird, welches aus der Gruppe ausgewählt ist, die aus Methanol, Ethanol, Dioxan, Tetrahydrofuran und 1,2-Dimethoxyethan besteht.

**8.** Verwendung eines Ascorbinsäurederivates, das durch die allgemeine Formel (IA) dargestellt wird:

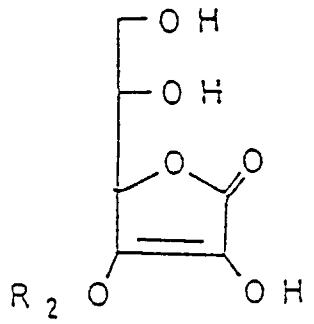

(IA),

worin $R_2$ eine Gruppe ist, welche aus der Gruppe ausgewählt ist, die aus einer einen heterocyclischen Ring enthaltenden $(C_1$-$C_5)$Alkylgruppe besteht, welche durch die allgemeinen Formeln dargestellt wird:

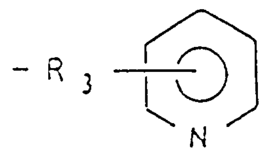

,

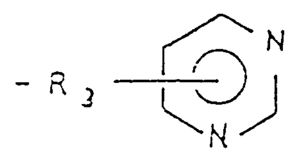

oder

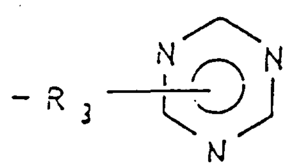

,

worin $R_3$ eine $(C_1$-$C_5)$Alkylengruppe, eine $(C_1$-$C_5)$Alkylcarbonyl-$(C_1$-$C_5)$alkylgruppe, eine $(C_6$-$C_{12})$-Arylcarbonyl-$(C_1$-$C_5)$alkylgruppe, eine $(C_7$-$C_{22})$Alkoxycarbonyl-$(C_1$-$C_5)$alkylgruppe, welche die $(C_7$-$C_{22})$-Alkoxygruppe endständig enthält, eine $(C_1$-$C_{12})$Alkylgruppe, eine $(C_7$-$C_{12})$Aralkylgruppe und eine Hydroxycarbonyl-$(C_1$-$C_{22})$alkylgruppe darstellt, als Antioxidans für Nahrungsmittel, als Verschönerungs- oder Bleichungskosmetikum oder als Antioxidans zum Eliminieren von Radikalen.

**9.** Verwendung gemäß Anspruch 8, wobei die genannte einen heterocyclischen Ring enthaltende $(C_1$-$C_5)$-Alkylgruppe aus der Gruppe ausgewählt ist, die aus Pyridylmethyl, Pyrimidylmethyl und Triazylmethyl besteht.

**10.** Verwendung gemäß Anspruch 8, wobei die genannte $(C_1$-$C_5)$Alkylcarbonyl-$(C_1$-$C_5)$Alkylgruppe durch die allgemeine Formel dargestellt wird:

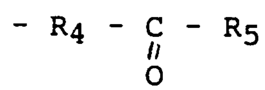

,

worin $R_4$ eine $(C_1$-$C_5)$Alkylengruppe und $R_5$ eine $(C_1$-$C_5)$Alkylgruppe darstellt.

**11.** Verwendung gemäß Anspruch 10, wobei die genannte $(C_1$-$C_5)$Akylcarbonyl-$(C_1$-$C_5)$alkylgruppe aus einer Gruppe ausgewählt ist, die aus $-CH_2CO$-$CH_3$ und $-CH_2$-$CO$-$C_2H_5$ besteht.

**12.** Verwendung gemäß Anspruch 8, wobei die genannte $(C_6$-$C_{12})$Arylcarbonyl-$(C_1$-$C_5)$alkylgruppe durch die allgemeine Formel dargestellt wird:

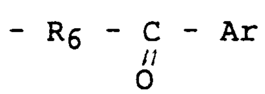

,

worin $R_6$ eine $(C_1$-$C_5)$Alkylengruppe und Ar eine $(C_6$-$C_{12})$Arylgruppe darstellt.

**13.** Verwendung gemäß Anspruch 12, wobei die genannte ($C_6$-$C_{12}$)Arylcarbonyl-($C_1$-$C_5$)Alkylgruppe $-CH_2CO-C_6H_5$ ist.

**14.** Verwendung gemäß Anspruch 8, wobei die genannte ($C_7$-$C_{22}$)Alkoxycarbonyl-($C_1$-$C_5$)alkylgruppe, welche die ($C_7$-$C_{22}$)Alkoxygruppe endständig enthält, durch die allgemeine Formel dargestellt wird:

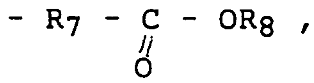

worin $R_7$ eine ($C_1$-$C_5$)Alkylengruppe darstellt, welche gegebenenfalls eine verzweigte Kette hat, und $R_8$ eine ($C_7$-$C_{22}$)Alkylgruppe ist.

**15.** Verwendung gemäß Anspruch 14, wobei $R_8$ eine ($C_7$-$C_{22}$)Alkylgruppe ist.

**16.** Verwendung gemäß Anspruch 15, wobei die genannte ($C_1$-$C_{22}$)Alkoxycarbonyl-($C_1$-$C_5$)alkylgruppe $-CH_2-COO-n-C_{10}H_{21}$ ist.

**17.** Verwendung gemäß Anspruch 8, wobei die genannte ($C_1$-$C_5$)Alkylgruppe eine gerad- oder verzweigtkettige Alkylgruppe ist.

**18.** Verwendung gemäß Anspruch 8, wobei der Alkylanteil der genannten Hydroxycarbonyl-($C_1$-$C_{22}$)-alkylgruppe eine gerad- oder verzweigtkettige Alkylgruppe ist.

**19.** Verwendung gemäß Anspruch 8, wobei der Alkylanteil der genannten ($C_7$-$C_{22}$)Aralkylgruppe eine gerad- oder verzweigtkettige Alkylgruppe ist.

**Patentansprüche für folgenden Vertragsstaat : ES**

**1.** Ein Verfahren zur Herstellung eines Ascorbinsäurederivates, welches durch die allgemeine Formel (Ia) dargestellt wird:

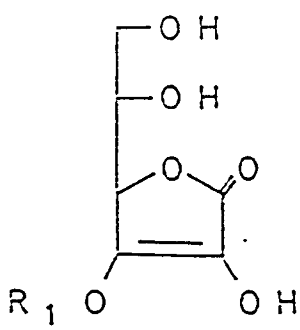

(Ia),

worin $R_1$ ausgewählt ist aus der Gruppe, welche aus einer einen heterocyclischen Ring enthaltenden ($C_1$-$C_5$)Alkylgruppe besteht, welche durch die allgemeine Formel dargestellt wird:

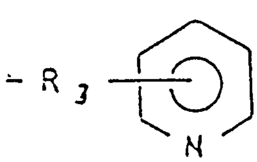

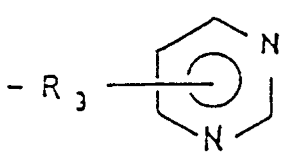

oder

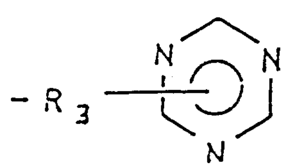

,

worin $R_3$ eine ($C_1$-$C_5$)Alkylengruppe und eine ($C_7$-$C_{22}$)Alkoxycarbonyl-($C_1$-$C_5$)alkylgruppe darstellt, welche die ($C_7$-$C_{22}$)Alkoxygruppe endständig enthält, wobei dieses Verfahren die Behandlung einer

Verbindung, welche durch die allgemeine Formel (II) dargestellt wird:

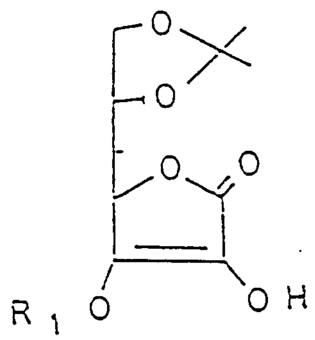

(II),

worin $R_1$ dieselbe Bedeutung wie oben hat, mit einer Säure einschließt, um den Dioxolanring in der genannten Verbindung aufzuspalten und eine vic-Glykolgruppe zu bilden.

**2.** Ein Verfahren gemäß Anspruch 1, wobei die genannte, einen heterocyclischen Ring enthaltende ($C_1$-$C_5$)Alkylgruppe aus einer Gruppe ausgewählt ist, die aus Pyridylmethyl, Pyrimidylmethyl und Triazylmethyl besteht.

**3.** Ein Verfahren gemäß Anspruch 1, wobei die genannte Alkoxycarbonylalkylgruppe durch die allgemeine Formel dargestellt wird:

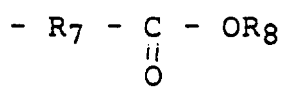

,

worin $R_7$ eine ($C_1$-$C_5$)Alkylengruppe darstellt, welche gegebenenfalls eine verzweigte Kette hat, und $R_8$ eine ($C_7$-$C_{22}$)Alkylgruppe ist.

**4.** Verfahren gemäß Anspruch 3, wobei die genannte Alkoxycarbonylalkylgruppe $-CH_2-COO-n-C_{10}H_{21}$ ist.

**5.** Ein Verfahren gemäß Anspruch 1, wobei die genannte Säure aus einer Gruppe ausgewählt wird, welche aus Salzsäure, Essigsäure, Schwefelsäure, p-Toluolsulfonsäure, Methansulfonsäure und Camphersulfonsäure besteht.

**6.** Ein Verfahren gemäß Anspruch 1, wobei die Reaktion bevorzugt in mindestens einem organischen Lösungsmittel durchgeführt wird, welches aus der Gruppe ausgewählt ist, die aus Methanol, Ethanol, Dioxan, Tetrahydrofuran und 1,2-Dimethoxyethan besteht.

**7.** Ein Verfahren zur Herstellung einer Zusammensetzung, welche eingesetzt werden kann als Antioxidans für Nahrungsmittel, als Verschönerungs- oder Bleichungskosmetikum oder als Antioxidans zum Eliminieren von Radikalen, worin ein Ascorbinsäurederivat, das durch die allgemeine Formel (IA) dargestellt wird:

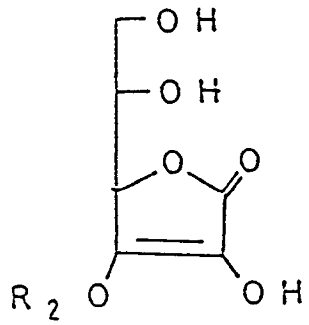

(IA),

worin $R_2$ eine Gruppe ist, welche aus der Gruppe ausgewählt ist, die aus einer einen heterocyclischen Ring enthaltenden ($C_1$-$C_5$)Alkylgruppe besteht, welche durch die allgemeinen Formeln dargestellt wird:

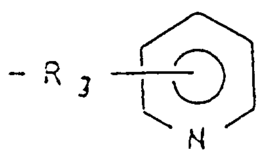

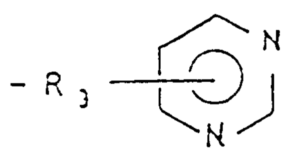

oder

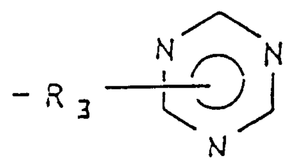

,

worin R3 eine ($C_1$-$C_5$)Alkylengruppe, eine ($C_1$-$C_5$)Alkylcarbonyl-($C_1$-$C_5$)alkylgruppe, eine ($C_6$-$C_{12}$)-Arylcarbonyl-($C_1$-$C_5$)alkylgruppe, eine ($C_7$-$C_{22}$)Alkoxycarbonyl-($C_1$-$C_5$)alkylgruppe, welche die ($C_7$-$C_{22}$)-Alkoxygruppe endständig enthält, eine ($C_1$-$C_{12}$)Alkylgruppe, eine ($C_7$-$C_{12}$)Aralkylgruppe und eine Hydroxycarbonyl-($C_1$-$C_{22}$)alkylgruppe darstellt, mit brauchbaren Zusätzen gemischt wird.

**8.** Ein Verfahren gemäß Anspruch 7, wobei die einen heterocyclischen Ring enthaltende ($C_1$-$C_5$)-Alkylgruppe ausgewählt ist aus der Gruppe, welche aus Pyridylmethyl, Pyrimidylmethyl und Triazylmethyl besteht.

**9.** Ein Verfahren gemäß Anspruch 7, wobei die genannte ($C_1$-$C_5$)Alkylcarbonyl-($C_1$-$C_5$)alkylgruppe durch die allgemeine Formel dargestellt wird:

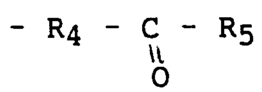

,

worin $R_4$ eine ($C_1$-$C_5$)Alkylengruppe und $R_5$ eine ($C_1$-$C_5$)Alkylgruppe darstellt.

**10.** Ein Verfahren gemäß Anspruch 9, wobei die genannte ($C_1$-$C_5$)Akylcarbonyl-($C_1$-$C_5$)alkylgruppe ausgewählt ist aus der Gruppe, welche aus $-CH_2CO-CH_3$ und $-CH_2-CO-C_2H_5$ besteht.

**11.** Ein Verfahren gemäß Anspruch 7, wobei die genannte ($C_6$-$C_{12}$)Arylcarbonyl-($C_1$-$C_5$)Alkylgruppe durch die allgemeine Formel dargestellt wird:

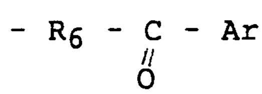

,

worin $R_6$ eine ($C_1$-$C_5$)Alkylengruppe und Ar eine ($C_6$-$C_{12}$)Arylgruppe darstellt.

**12.** Ein Verfahren gemäß Anspruch 11, wobei die genannte $(C_6\text{-}C_{12})$Arylcarbonyl-$(C_1\text{-}C_5)$alkylgruppe $-CH_2CO\text{-}C_6H_5$ ist.

**13.** Ein Verfahren gemäß Anspruch 7, wobei die genannte $(C_7\text{-}C_{22})$Alkoxycarbonyl-$(C_1\text{-}C_5)$alkylgruppe, welche die $(C_7\text{-}C_{22})$Alkoxygruppe endständig enthält und welche durch die allgemeine Formel dargestellt wird:

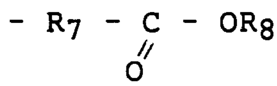 ,

worin $R_7$ eine $(C_1\text{-}C_5)$Alkylengruppe darstellt, welche gegebenenfalls eine verzweigte Kette hat, und $R_8$ eine $(C_7\text{-}C_{22})$Alkylgruppe ist.

**14.** Ein Verfahren gemäß Anspruch 13, wobei $R_8$ eine $(C_7\text{-}C_{22})$Alkylgruppe ist.

**15.** Ein Verfahren gemäß Anspruch 14, wobei die genannte $(C_1\text{-}C_{22})$Alkoxycarbonyl-$(C_1\text{-}C_5)$alkylgruppe $-CH_2\text{-}COO\text{-}n\text{-}C_{10}H_{21}$ ist.

**16.** Ein Verfahren gemäß Anspruch 7, wobei die genannte $(C_1\text{-}C_5)$Alkylgruppe eine gerad- oder verzweigtkettige Alkylgruppe ist.

**17.** Ein Verfahren gemäß Anspruch 7, wobei der Alkylanteil der genannten Hydroxycarbonyl$(C_1\text{-}C_{22})$-alkylgruppe eine gerad- oder verzweigtkettige Alkylgruppe ist.

**18.** Ein Verfahren gemäß Anspruch 7, wobei der Alkylanteil der genannten $(C_7\text{-}C_{12})$Aralkylgruppe eine gerad- oder verzweigtkettige Alkylgruppe ist.

## Revendications

### Revendications pour les Etats contractants suivants : AT, BE, CH, DE, FR, GB, GR, IT, LI, LU, NL, SE

**1.** Dérivé d'acide ascorbique représente par la formule générale (Ia) :

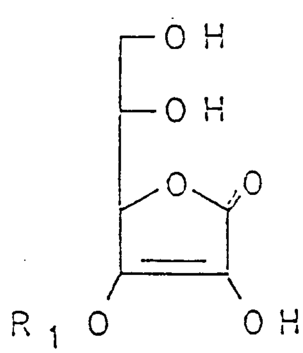

( I a )

dans laquelle $R_1$ est choisi dans le groupe constitué d'un groupe alkyl (en $C_1$ à $C_5$) contenant un noyau hétérocyclique, représenté par la formule générale :

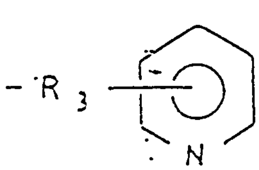 , 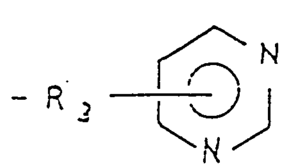 ou 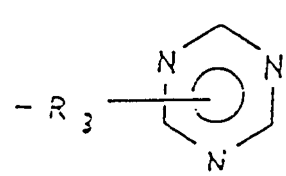

dans laquelle $R_3$ représente un groupe alkylène en $C_1$ à $C_5$, et un groupe alkyl (en $C_1$ à $C_5$) - alcoxy

en ($C_7$ à $C_{22}$)carbonyle contenant le groupe alcoxy (en $C_7$ à $C_{22}$) terminal.

**2.** Dérivé d'acide ascorbique selon la revendication 1, dans lequel ledit groupe alkyl (en $C_1$ à $C_5$) contenant un noyau hétérocyclique est choisi dans le groupe constitué des groupes pyridylméthyle, pyrimidylméthyle et triazylméthyle.

**3.** Dérivé d'acide ascorbique selon la revendication 1, dans lequel ledit groupe alcoxycarbonylalkyle est représenté par la formule générale :

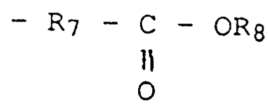

dans laquelle $R_7$ représente un groupe alkylène en $C_1$ à $C_5$ qui peut avoir en option une chaîne ramifiée et $R_8$ est un groupe alkyle en $C_7$ à $C_{22}$.

**4.** Dérivé d'acide ascorbique selon la revendication 3, dans lequel ledit groupe alcoxycarbonylalkyle est $-CH_2-COO-n-C_{10}H_{21}$.

**5.** Procédé pour préparer un dérivé d'acide ascorbique représenté par la formule générale (Ia) :

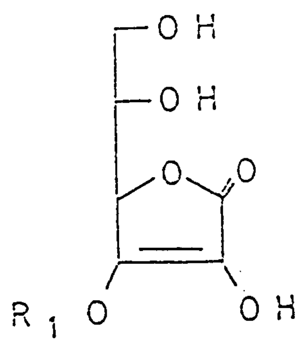

(Ia)

dans laquelle $R_1$ est choisi dans le groupe constitué d'un groupe alkyl (en $C_1$ à $C_5$) contenant un noyau hétérocyclique, représenté par la formule générale :

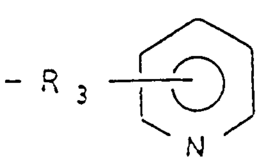 , 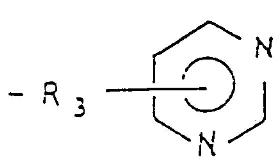 ou 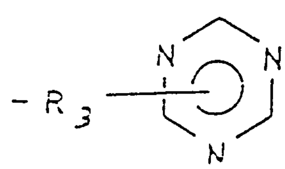

dans laquelle $R_3$ représente un groupe alkylène en $C_1$ à $C_5$, et un groupe alkyl (en $C_1$ à $C_5$) - alcoxy (en $C_7$ à $C_{22}$)carbonyle contenant le groupe alcoxy (en $C_7$ à $C_{22}$) terminal, qui comprend le traitement d'un composé représenté par la formule générale (II) :

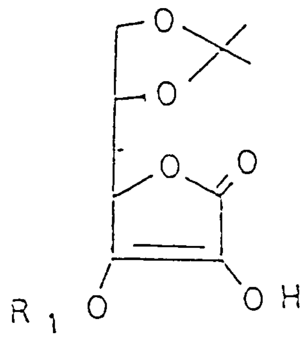

( II )

dans laquelle $R_1$ a la même signification que ci-dessus, avec un acide pour couper le noyau dioxolane en ledit composé et former un groupe vic-glycol.

6. Procédé selon la revendication 5, dans lequel ledit acide est choisi dans le groupe constitué de l'acide chlorhydrique, de l'acide acétique, de l'acide sulfurique, de l'acide p-toluènesulfonique, de l'acide méthanesulfonique et de l'acide camphresulfonique.

7. Procédé selon la revendication 5, dans lequel la réaction est effectuée dans au moins un solvant organique choisi dans le groupe constitué du méthanol, de l'éthanol, du dioxane, du tétrahydrofurane et du 1,2-diméthoxyéthane.

8. Utilisation d'un dérivé d'acide ascorbique représenté par la formule générale (IA) :

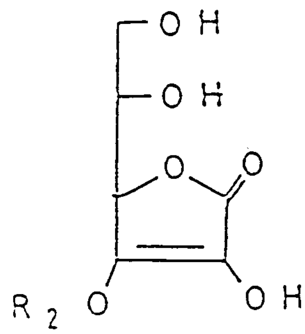

( I A )

dans laquelle $R_2$ est un groupe choisi dans le groupe constitué d'un groupe alkyl (en $C_1$ à $C_5$) contenant un noyau hétérocyclique, représenté par la formule générale:

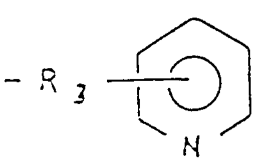 , 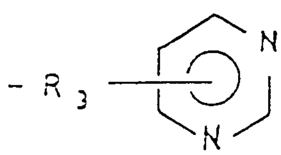 ou 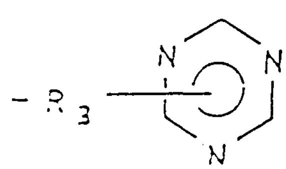

dans laquelle $R_3$ représente un groupe alkylène en $C_1$ à $C_5$, un groupe alkyl (en $C_1$ à $C_5$) - alkyl (en $C_1$ à $C_5$) carbonyle, un groupe alkyl (en $C_1$ à $C_5$) - aryl (en $C_6$ à $C_{12}$)carbonyle, un groupe alkyl (en $C_1$ à $C_5$) - alcoxy (en $C_7$ à $C_{22}$)carbonyle contenant le groupe alcoxy (en $C_7$ à $C_{22}$) terminal, un groupe alkyl (en $C_1$ à $C_{12}$), un groupe aralkyl (en $C_7$ à $C_{12}$) et un groupe alkyl (en $C_1$ à $C_{22}$) hydroxycarbonyle comme anti-oxydant dans les aliments, produits d'embellissement et d'éclaircissage ou antioxydant pour l'élimination des radicaux.

**9.** Utilisation selon la revendication 8, dans laquelle ledit groupe alkyl (en $C_1$ à $C_5$) contenant un noyau hétérocyclique est choisi dans le groupe constitué des groupes pyridylméthyle, pyrimidylméthyle et triazylméthyle.

**10.** Utilisation selon la revendication 8, dans laquelle ledit groupe alkyl (en $C_1$ à $C_5$) - alkyl (en $C_1$ à $C_5$) carbonyle est représenté par le formule générale :

$$-R_4-\underset{\underset{O}{\|}}{C}-R_5$$

dans laquelle $R_4$ représente un groupe alkylène (en $C_1$ à $C_5$) et $R_5$ représente un groupe alkyl (en $C_1$ à $C_5$).

**11.** Utilisation selon la revendication 10, dans laquelle ledit groupe alkyl (en $C_1$ à $C_5$) - alkyl (en $C_1$ à $C_5$)-carbonyle est choisi dans le groupe constitué de $-CH_2CO-CH_3$ et $-CH_2CO-C_2H_5$.

**12.** Utilisation selon la revendication 8, dans laquelle ledit groupe alkyl (en $C_1$ à $C_5$) - aryl (en $C_6$ à $C_{12}$)-carbonyle est représenté par la formule générale:

$$-R_6-\underset{\underset{O}{\|}}{C}-Ar$$

dans laquelle $R_6$ représente un groupe alkylène (en $C_1$ à $C_5$) et Ar est un groupe aryl (en $C_6$ à $C_{12}$).

**13.** Utilisation selon la revendication 12, dans laquelle ledit groupe alkyl (en $C_1$ à $C_5$) - aryl (en $C_6$ à $C_{12}$)-carbonyle est $-CH_2CO-C_6H_5$.

**14.** Utilisation selon la revendication 8, dans laquelle ledit groupe alkyl (en $C_1$ à $C_5$) - alcoxy (en $C_7$ à $C_{22}$)-carbonyle contenant le groupe alcoxy (en $C_7$ à $C_{22}$) terminal est représenté par la formule générale :

$$-R_7-\underset{\underset{O}{\|}}{C}-OR_8$$

dans laquelle $R_7$ représente un groupe alkylène en $C_1$ à $C_5$ qui peut avoir en option une chaîne ramifiée et $R_8$ est un groupe alkyle en $C_7$ à $C_{22}$.

**15.** Utilisation selon la revendication 14, dans laquelle $R_8$ est un groupe alkyle en $C_7$ à $C_{22}$.

**16.** Utilisation selon la revendication 15, dans laquelle ledit groupe alkyl (en $C_1$ à $C_5$) - alcoxy (en $C_7$ à $C_{22}$)carbonyle est $-CH_2-COO-n-C_{10}H_{21}$.

**17.** Utilisation selon la revendication 8, dans laquelle ledit groupe alkyle en $C_1$ à $C_5$ est un groupe alkyle linéaire ou ramifié.

**18.** Utilisation selon la revendication 8, dans laquelle la partie alkyle dudit groupe alkyl (en $C_1$ à $C_{22}$)-hydroxycarbonyle est un groupe alkyle linéaire ou ramifié.

**19.** Utilisation selon la revendication 8, dans laquelle le partie alkyle dudit groupe aralkyle en $C_7$ à $C_{12}$ est un groupe alkyle linéaire ou ramifié.

**Revendications pour l'Etat contractant suivant : ES**

1. Procédé pour préparer un dérivé d'acide ascorbique représenté par la formule générale (Ia):

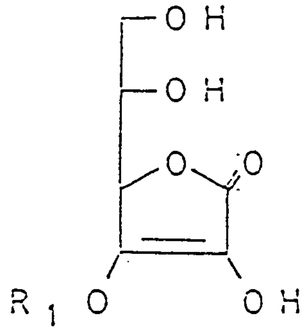

(I a)

dans laquelle $R_1$ est choisi dans le groupe constitué d'un groupe alkyl (en $C_1$ à $C_5$) contenant un noyau hétérocyclique, représenté par la formule générale :

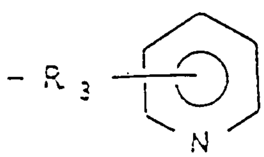

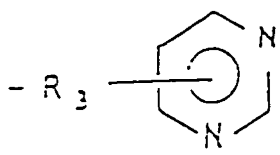

ou

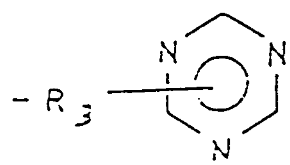

dans laquelle $R_3$ représente un groupe alkylène en $C_1$ à $C_5$, et un groupe alkyl (en $C_1$ à $C_5$) - alcoxy (en $C_7$ à $C_{22}$)carbonyle contenant le groupe alcoxy (en $C_7$ à $C_{22}$) terminal, qui comprend le traitement d'un composé représenté par la formule générale (II) :

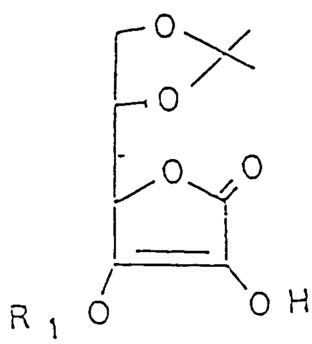

(II)

dans laquelle $R_1$ a la même signification que ci-dessus, avec un acide pour couper le noyau dioxolane en ledit composé et former un groupe vic-glycol.

2. Procédé selon la revendication 1, dans lequel ledit groupe alkyl (en $C_1$ à $C_5$) contenant un noyau hétérocyclique est choisi dans le groupe constitué des groupes pyridylméthyle, pyrimidylméthyle et triazylméthyle.

3. Procédé selon la revendication 1, dans lequel ledit groupe alcoxycarbonylalkyle est représenté par la formule générale :

$$-R_7-\underset{\underset{O}{\parallel}}{C}-OR_8$$

dans laquelle $R_7$ représente un groupe alkylène en $C_1$ à $C_5$ qui peut en option comporter une chaîne ramifiée et $R_8$ est un groupe alkyl (en $C_7$ à $C_{22}$).

**4.** Procédé selon la revendication 3, dans lequel ledit groupe alcoxycarbonylalkyle est $-CH_2-COO-n-C_{10}H_{21}$.

**5.** Procédé selon la revendication 1, dans lequel ledit acide est choisi dans le groupe constitué de l'acide chlorhydrique, de l'acide acétique, de l'acide sulfurique, de l'acide p-toluènesulfonique, de l'acide méthanesulfonique et de l'acide camphresulfonique.

**6.** Procédé selon la revendication 1, dans lequel la réaction est effectuée dans au moins un solvant organique choisi dans le groupe constitué du méthanol, de l'éthanol, du dioxane, du tétrahydrofurane et du 1,2-diméthoxyéthane.

**7.** Procédé pour préparer une composition utilisée comme anti-oxydant des aliments, comme cosmétiques d'embellissement et d'éclaircissage ou comme anti-oxydant pour l'élimination des radicaux, dans lequel un dérivé d'acide ascorbique représenté par la formule générale (IA):

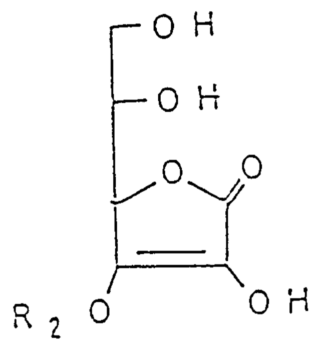

(IA)

dans laquelle $R_2$ est un groupe choisi dans le groupe constitué d'un groupe alkyl (en $C_1$ à $C_5$) contenant un noyau hétérocyclique, représenté par la formule générale:

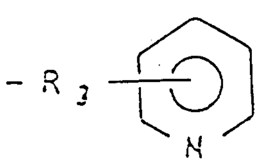 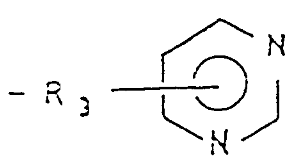 ou 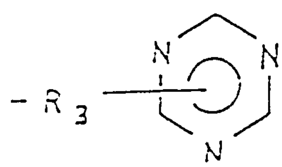

dans laquelle $R_3$ représente un groupe alkylène en $C_1$ à $C_5$, un groupe alkyl (en $C_1$ à $C_5$) - alkyl (en $C_1$ à $C_5$) carbonyle, un groupe alkyl (en $C_1$ à $C_5$) - aryl (en $C_6$ à $C_{12}$)carbonyle, un groupe alkyl (en $C_1$ à $C_5$) - alcoxy (en $C_7$ à $C_{22}$)carbonyle contenant le groupe alcoxy (en $C_7$ à $C_{22}$) terminal, un groupe alkyl (en $C_1$ à $C_{12}$), un groupe aralkyl (en $C_7$ à $C_{12}$) et un groupe alkyl (en $C_1$ à $C_{22}$) hydroxycarbonyle, est mélangé avec des additifs acceptables.

**8.** Procédé selon la revendication 7, dans lequel ledit groupe alkyl (en $C_1$ à $C_5$) contenant un noyau hétérocyclique est choisi dans le groupe constitué des groupes pyridylméthyle, pyrimidylméthyle et triazylméthyle.

**9.** Procédé selon la revendication 7, dans lequel ledit groupe alkyl (en $C_1$ à $C_5$) - alkyl (en $C_1$ à $C_5$) carbonyle est représenté par la formule générale :

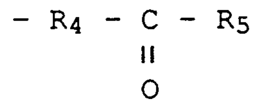

dans laquelle $R_4$ représente un groupe alkylène (en $C_1$ à $C_5$) et $R_5$ représente un groupe alkyl (en $C_1$ à $C_5$).

**10.** Procédé selon la revendication 9, dans lequel ledit groupe alkyl (en $C_1$ à $C_5$) - alkyl (en $C_1$ à $C_5$)-carbonyle est choisi dans le groupe constitué de $-CH_2CO-CH_3$ et $-CH_2CO-C_2H_5$.

**11.** Procédé selon la revendication 7, dans lequel ledit groupe alkyl (en $C_1$ à $C_5$) - aryl (en $C_6$ à $C_{12}$)-carbonyle est représenté par la formule générale:

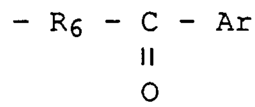

dans laquelle $R_6$ représente un groupe alkylène (en $C_1$ à $C_5$) et Ar est un groupe aryl (en $C_6$ à $C_{12}$).

**12.** Procédé selon la revendication 11, dans lequel ledit groupe alkyl (en $C_1$ à $C_5$) - aryl (en $C_6$ à $C_{12}$) carbonyle est $-CH_2CO-C_6H_5$.

**13.** Procédé selon la revendication 7, dans lequelle ledit groupe alkyl (en $C_1$ à $C_5$) - alcoxy (en $C_7$ à $C_{22}$) carbonyle contenant le groupe alcoxy (en $C_7$ à $C_{22}$) terminal est représenté par la formule générale :

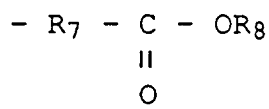

dans laquelle $R_7$ représente un groupe alkylène (en $C_1$ à $C_5$) qui peut en option avoir une chaîne ramifiée et $R_8$ est un groupe alkyl (en $C_7$ à $C_{22}$).

**14.** Procédé selon la revendication 13, dans lequel $R_8$ est un groupe alkyl (en $C_7$ à $C_{22}$).

**15.** Procédé selon la revendication 14, dans lequel ledit groupe alkyl (en $C_1$ à $C_5$) - alcoxy (en $C_7$ à $C_{22}$)-carbonyle est $-CH_2-COO-n-C_{10}H_{21}$.

**16.** Procédé selon la revendication 7, dans lequel ledit groupe alkyl (en $C_1$ à $C_5$) est un groupe alkyle linéaire ou ramifié.

**17.** Procédé selon la revendication 7, dans lequel la partie alkyle dudit groupe alkyl (en $C_1$ à $C_{22}$)-hydroxycarbonyle est un groupe alkyle linéaire ou ramifié.

**18.** Procédé selon la revendication 7, dans lequel le partie alkyle dudit groupe aralkyl (en $C_7$ à $C_{12}$) est un groupe alkyle linéaire ou ramifié.